# EUROPEAN PATENT SPECIFICATION

(11) **EP 2 247 572 B1**
(45) Date of publication and mention of the grant of the patent: **03.08.2011**
(21) Application number: 09713008.2
(22) Date of filing: 19.02.2009
(51) Int. Cl.: C07C 241/02, C07C 243/40

(54) **A ONE-POT PROCESS FOR PREPARING 3-(2,2,2-TRIMETHYLHYDRAZINIUM)PROPIONATE DIHYDRATE**
EIN-TOPF-VERFAHREN ZUR HERSTELLUNG VON 3-(2,2,2-TRIMETHYLHYDRAZINIUM)PROPIONAT-DIHYDRAT
PROCÉDÉ DE PRÉPARATION EN ENCEINTE UNIQUE DE DIHYDRATE DE 3-(2,2,2-TRIMÉTHYLHYDRAZINIUM)-PROPIONATE

(30) Priority: 19.02.2008 LV 080022; 19.02.2008 LV 080023
(43) Date of publication of application: 10.11.2010
(73) Proprietor: Grindeks, a joint stock company, Riga 1057 (LV)
(72) Inventor: KALVINS, Ivars, LV-15052 Ikskile (LV); OSVALDS, Pugovics, LV-1004 Riga (LV); CERNOBROVIJS, Aleksandrs, LV-2114 Olaine (LV); IEVINA, Agnija, LV-1004 Riga (LV); LEBEDEVS, Antons, LV-3600 Ventspils (LV)
(86) International application number: PCT/EP2009/051996
(87) International publication number: WO 2009/103773

(56) References cited:
- WO-A-2005/012233
- US-A- 4 481 218
- GILLER S.A. ET AL,.: CHEMISTRY OF HETEROCYCLIC COMPOUNDS, vol. 11, no. 12, 1975, pages 1378-1382, XP002534780

## Description

### Technical Field

The present invention relates to one-pot process for preparing 3-(2,2,2-trimethylhydrazinium)propionate dihydrate (international non-proprietary name - "Meldonium") from 3-(2,2-dimethylhydrazino)propionate esters having general formula (I). 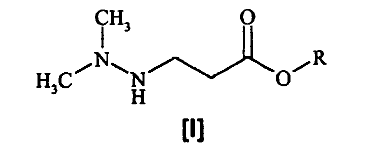 wherein R represents CH₃, C₂H₅, C₃H₇, i-C₃H₇, C₄H₉, i-C₄H₉ and C₆H₅CH₂ and from the corresponding acid (R represents H).

### Background Art

3-(2,2,2-Trimethylhydrazinium)propionate dihydrate is known for its cardioprotective properties under the International Non-proprietary Name of Meldonium. A number of methods for the preparation of 3-(2,2,2-trimethylhydrazinium)propionate dihydrate are known.

Generally, the process involves 1,1-dimethylhydrazine reaction with acrylic acid esters leading to 3-(2,2-dimethylhydrazino)propionate esters, further alkylated with a methyl halide or dimethylsulphate to give the appropriate methyl-3-(2,2,2-trimethylhydrazinium)propionate halogenide or methylsulphate, which are then hydrolyzed and deionized.

A standard alkaline hydrolysis method of carbonic and sulphuric acid esters in case of the alkyl-3-(2,2,2-trimethylhydrazinium)propionate salts was not successfully realized till now because of the problems of separation of 3-(2,2,2- trimethylhydrazinium) propionate dihydrate and the resulting inorganic salts. It is known that 3-(2,2,2-trimethylhydrazinium)propionate dihydrate forms relatively stable double salts of the variable composition (SU849724).

WO 2008/028514 A (SILVA JORGE) 13.03.2008, disclosed a method of producing the 3-(2,2,2-trimethylhydrazinium) propionate dihydrate by hydrolyzing 3-(2,2,2-trimethylhydrazinium)propionate halide or methyl sulphate esters under acidic conditions, catalyzed by HCl, sulphuric acid, phosphoric acid etc., followed by neutralization by an appropriate inorganic base (for example, sodium, potassium, calcium or magnesium hydroxide or another appropriate base, for example sodium, potassium, lithium or cesium carbonate or bicarbonate etc.) and the double salts thus obtained can be separated by the invented process using saturation of the solution with carbon dioxide or sulphur dioxide.

US4481218 discloses a preparation of 3-(2,2,2-trimethylhydrazinium)propionate dihydrate, which comprises passage through a column with a strongly basic ion exchange resin.

### Disclosure of Invention

We have unexpectedly found, that preparation of the 3-(2,2,2-trimethylhydrazinium)propionate dihydrate is possible to carry out in a one-pot process using as a starting material 3-(2,2-dimethylhydrazino)propionate esters of the general formula (I), 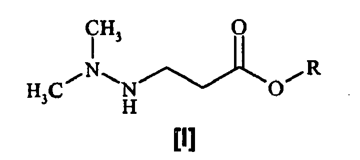 wherein R represents CH₃, C₂H₅, C₃H₇, i-C₃H₇, C₄H₉, i-C₄H₉ and C₆H₅CH₂, hydrolyzing the 3-(2,2-dimethylhydrazino)propionate esters of the formula (I) in a solvent, followed by the reaction with dimethyl carbonate (DMC). Surprisingly, we have also found that a direct one-pot synthesis of the 3-(2,2,2-trimethylhydrazinium)propionate dihydrate by the hydrolysis of the 3-(2,2-dimethylhydrazino)propionate esters of the formula (I) and simultaneous reaction of the formed intermediate with DMC can also be performed with high yields of the desired product.

According to the present invention, the solvent for the invented process is selected from the group, comprising water and water-containing solvents e. g. lower alcohols, acetone, ethyl acetate, acetonitrile, dioxane, dimethylformamide, dimethylsulphoxide, alkylketones and mixtures thereof.

In one embodiment of the current invention, hydrolysis of 3-(2,2-dimethylhydrazino)propionate esters of the formula I was carried out at 70-100°C in water until complete conversion of the starting ester to the corresponding acid, controlled chromatographically. After the conversion was completed, the process was continued, without isolation of the intermediates, by adding to reaction mixture dimethyl carbonate and methanol. Reaction was continued at the same temperature until the conversion was completed (with chromatographic control), then methanol, dimethyl carbonate and water were removed by distillation at a reduced pressure. The distillation residue was treated with hot isopropyl alcohol, filtered and dried, yielding of 90-98% of the desired 3-(2,2,2-trimethylhydrazinium)propionate dihydrate.

In another embodiment of the current invention, 3-(2,2-dimethylhydrazino)propionate ester of the formula I was dissolved in water, and methanol and dimethyl carbonate were added to this solution. The reaction mixture was heated at 80-100°C with chromatographic control until the full conversion of the starting ester. After removal of the solvents the residue was treated with hot isopropyl alcohol, filtered and dried, yielding of 90-98% of the desired 3-(2,2,2-trimethylhydrazinium)propionate dihydrate. Thus, according to the current invention, it is possible to obtain 3-(2,2,2-trimethylhydrazinium)propionate dihydrate from methyl-3-(2,2-dimethylhydrazino)propionate in a one-pot reaction, without isolation of any intermediates and significantly increasing the reaction rate and total yield of the final product.

In yet another embodiment of the current invention, 3-(2,2,2-trimethylhydrazinium)propionate dihydrate can also be obtained from the 3-(2,2-dimethylhydrazino)propionic acid, which forms as an intermediate in the invented process, reacting the 3-(2,2-dimethylhydrazino)propionic acid with DMC in a solvent, followed by evaporation of the liquids, treatment of the residue with hot isopropyl alcohol, filtration and drying of the crude 3-(2,2,2-trimethylhydrazinium)propionate dihydrate, with 95-98% yields of the desired product. Methods of the current invention overcomes the disadvantages of the method disclosed in WO 2008/028514 A (SILVA JORGE) 13.03.2008 by presenting the one-pot process for obtaining 3-(2,2,2-trimethylhydrazinium)propionate dihydrate from 3-(2,2-dimethylhydrazino)propionate esters and/or 3-(2,2-dimethylhydrazino)propionic acid, reducing the number of reaction steps and eliminating the problem of formation of the double salts. Thus, the current invention provide for an inexpensive and convenient large scale manufacturing process of 3-(2,2,2-trimethylhydrazinium)propionate dihydrate under use of inexpensive and available reagents with high yields and purity of the final product.

The present invention will be described in more detail by referring to the following nonlimiting examples. The scope of the invention should not be limited to the working examples, which are for demonstration purposes. One skilled in the art can practice the invention based on the disclosures in the present patent application.

### Example 1

Preparation of 3-(2,2,2-trimethylhydrazinium)propionate dihydrate Methyl-3-(2,2-dimethylhydrazino)propionate (146 g, 1 mol) was dissolved in water (290 mL) and heated under inert atmosphere at 90-100°C until complete conversion of starting ester to corresponding acid (controlled by HPLC). Methanol (150 ml), thereafter dimethyl carbonate (843 mL, 10 mol) was added to the reaction mixture. Reaction mixture was heated at 90°C in a reactor until the reaction was complete (controlled by HPLC). Methanol, dimethyl carbonate and water were removed by distillation at a reduced pressure. The distillation residue was dissolved in hot isopropyl alcohol (500 ml), and evaporated again. Obtained 3-(2,2,2-trimethylhydrazinium)propionate dihydrate was dried under reduced pressure. The yield was 172 g (94.4%).

### Example 2

Preparation of 3-(2,2,2-trimethylhydrazinium)propionate dihydrate Methyl-3-(2,2-dimethylhydrazino)propionate (146 g, 1 mol) was dissolved in water (270 mL), then methanol (170 ml) and dimethyl carbonate (843 mL, 10 mol) were added to the solution. Reaction mixture was heated at 95°C in a reactor until the reaction was complete (controlled by HPLC). Methanol, dimethyl carbonate and water were removed by distillation at a reduced pressure. The distillation residue was dissolved in hot isopropyl alcohol (500 ml), and evaporated again. Obtained 3-(2,2,2-trimethylhydrazinium)propionate dihydrate was dried under reduced pressure. The yield was 171 g (93.9%).

### Example 3

Preparation of 3-(2,2,2-trimethylhydrazinium)propionate dihydrate 3-(2,2-Dimethylhydrazino)propionic acid (132 g, 1 mol) was dissolved in water (180 mL), thereafter methanol (170 ml) and dimethyl carbonate (843 mL, 10 mol) were added to the reaction mixture. Reaction mixture was heated at 95°C in a reactor until the reaction was complete (controlled by HPLC).

Methanol, dimethyl carbonate and water were removed by distillation at a reduced pressure. The distillation residue dissolved in hot isopropyl alcohol (500 ml), and evaporated again. Obtained 3-(2,2,2-trimethylhydrazinium)propionate dihydrate was dried under reduced pressure. The yield was 175 g (96.0%).

## Claims

1. A one-pot process for preparing 3-(2,2,2-trimethylhydrazinium)propionate dihydrate from 3-(2,2-dimethylhydrazino)propionate esters, having general formula (I) 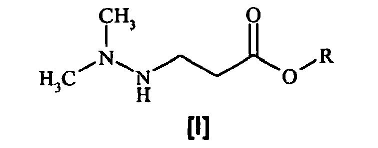 wherein R represents CH₃, C₂H₅, C₃H₇, i-C₃H₇, C₄H₉, i-C₄H₉ and C₆H₅CH₂, and from the 3-(2,2-dimethylhydrazino)propionic acid (R represents H),
by reaction with dimethyl carbonate in a solvent, selected from the group, comprising water and water-containing solvents, e. g. lower alcohols, acetone, ethyl acetate, acetonitrile, dioxane, dimethylformamide, dimethylsulphoxide, alkylketones and mixtures thereof and at a temperature in the range from 70-100°C.

2. A process according to the claim 1, wherein the dimethyl carbonate is added to the reaction mixture after the hydrolysis of the compound of formula (I) in a solvent is completed.

3. A process according to the claim 1, wherein the dimethyl carbonate is added to the reaction mixture together with the solvent.

4. A process according to the claims 1-3, wherein the solvent is water.

5. A process according to the claims 1-3, wherein the solvent is a mixture of water and methanol.

6. A process according to claims 1-3, wherein 3-(2,2-dimethylhydrazino)propionate ester of formula (I) is methyl-3-(2,2-dimethylhydrazino)propionate.

7. A process according to claims 1-3, wherein the compound of formula (I) is 3-(2,2-dimethylhydrazino)propionic acid.

8. A process according to claim 7, wherein the reaction is carried out at the temperatures in the range from 90-95°C.

## Patentansprüche

1. In einem Gefäß durchzuführendes Verfahren zur Gewinnung von 3-(2,2,2-trimethylhydrazin)Propionat-Dihydrat aus 3-(2,2-dimethylhydrazin)Propionatester, wenn die allgemeine Formel (I) 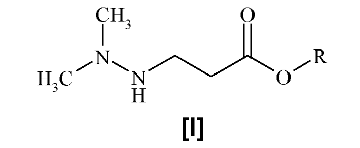 zugänglich ist, worin R sich auf CH₃, C₂H₅, C₃H₇, i-C₃H₇, C₄H₉, i-C₄H₉ und C₆H₅CH₂ bezieht, und aus 3-(2,2-dimethylhydrazin)Propionsäure (R bezieht sich auf H),
unter Anwendung der Reaktion mit Dimethylcarbonat in einem Lösungsmittel, das aus einer Gruppe gewählt wird, in die auch Wasser und lösungsmittelhaltiges Wasser fallen, das zum Beispiel niedere Alkohole, Aceton, Ethylacetat, Acetonitril, Dioxan, Dimethylformamid, Dimethylsulfoxid, Alkylketone und Mischungen aus diesen enthält, und bei Temperaturen zwischen 70 und 100°C.

2. Verfahren nach Anspruch 1, worin nach der Hydrolyse der Verbindung zu Lösungsmittel das Dimethylcarbonat der Reaktionsmischung gemäß Formel (I) zugegeben wird.

3. Verfahren nach Anspruch 1, worin das Dimethylcarbonat der Reaktionsmischung zusammen mit dem Lösungsmittel zugegeben wird.

4. Verfahren nach Anspruch 1-3, worin Wasser das Lösungsmittel ist.

5. Verfahren nach Anspruch 1-3, worin eine Mischung aus Wasser und Methanol das Lösungsmittel ist.

6. Verfahren nach Anspruch 1-3, worin Methyl-3-(2,2-dimethylhydrazin)Propionat der 3-(2,2-dimethylhydrazin)Propionatester gemäß Formel (I) ist.

7. Verfahren nach Anspruch 1-3, worin die 3-(2,2-dimethylhydrazin)Propionsäure die Verbindung gemäß Formel (I) ist.

8. Verfahren nach Anspruch 7, worin die Reaktion bei Temperaturen zwischen 90 und 95 °C durchgeführt wird.

## Revendications

1. Le processus doit être faits dans un récipient pour préparer le 3-(2,2,2-triméthyl hydrazine) propionate dihydrate des esters propionate 3-(2,2-dimethylhydrazino) si la formule générale est (I) 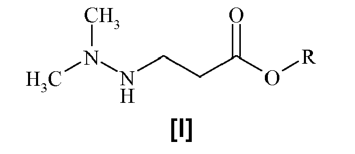 où R représente CH₃, C₂H₅, C₃H₇, i-C₃H₇, C₄H₉, i-C₄H₉ et C₆H₅CH₂, et de 3-(2,2-dimethylhydrazino) acide-propionique (R représente H),
en utilisant la réaction avec le dimethylcarbonate dans le solvant, choisi dans le groupe qui embrasse l'eau et les solvants aqueux, par ex., alcools inférieurs, acétone, acétate d'éthyle, acétonitrile, dioxane, dimethylformamide, diméthylsulfoxyde, alkylketones et leurs mélanges, et à la température dans les limites entre 70 et 100 °C.

2. Le processus conforme à la première revendication où le carbonate de diméthyle est ajouté au mélange de réaction après l'hydrolyse, selon la formule (I).

3. Le processus conforme à la première revendication où le dimethylcarbonate est ajouté au mélange de reaction ensemble avec le solvant.

4. Le processus conforme aux revendications 1 - 3 , où le solvant est l'eau.

5. Le processus conforme aux revendications 1 - 3 , où le solvant est le mélange de l'eau et de méthanol.

6. Le processus conforme aux revendications 1 - 3 , où l'ester propionate de la formule (I) 3-(2,2-dimetilhidrazino) est le méthyl-3-(2,2-diméthylhydrazine)propionate.

7. Le processus conforme aux revendications 1 - 3 , où le composé de formule (I) est 3-(2,2-dimethylhydrazino) acide-propionique.

8. Le processus conforme à la 7^{ième} revendication, où la réaction est faite à la température dans les limites entre 90 et 95 °C.
